# EUROPEAN PATENT SPECIFICATION

(11) **EP 2 812 385 B1**
(45) Date of publication and mention of the grant of the patent: **04.10.2017**
(21) Application number: 13747075.3
(22) Date of filing: 07.02.2013
(51) Int. Cl.: C08J 9/06, C08L 23/28, C08K 5/08, C08K 3/34, C08K 3/36

(54) **BLOWING AGENTS, FOAM PREMIXES AND FOAMS CONTAINING HALOGENATED OLEFIN BLOWING AGENT AND ABSORBENT**
BLASMITTEL, SCHAUMVORMISCHUNGEN UND SCHÄUME MIT HALOGENIERTEN OLEFINBLASMITTELN UND ABSORPTIONSMITTELN
AGENTS D'EXPANSION, PRÉMÉLANGES DE MOUSSE ET MOUSSES CONTENANT UN ABSORBANT ET UN AGENT D'EXPANSION À BASE D'OLÉFINE HALOGÉNÉE

(30) Priority: 10.02.2012 US 201261597410 P; 06.02.2013 US 201313760866
(43) Date of publication of application: 17.12.2014
(73) Proprietor: Honeywell International Inc., Morris Plains, NJ 07950 (US)
(72) Inventor: LING, Yiu Keung, Morristown, New Jersey 07962-2245 (US); WILLIAMS, David, J., Morristown, New Jersey 07962-2245 (US); BOGDAN, Mary, C., Morristown, New Jersey 07962-2245 (US)
(74) Representative: Crooks, Elizabeth Caroline
(86) International application number: PCT/US2013/025024
(87) International publication number: WO 2013/119731

(56) References cited:
- EP-A1- 0 341 210
- JP-A- H08 157 709
- KR-A- 20080 025 748
- US-A- 4 230 566
- US-A- 5 137 929
- US-A- 5 137 929
- US-A1- 2005 222 380
- US-A1- 2009 099 272

## Description

### FIELD OF THE INVENTION

The present invention pertains to polyurethane and polyisocyanurate foams and foam premixes, to blowing agents, catalyst systems, adsorbent materials, and methods for the preparation thereof.

### BACKGROUND OF THE INVENTION

Rigid to semi-rigid polyurethane or polyisocyanurate foams have utility in a wide variety of insulation applications including roofing systems, building panels, building envelope insulation, spray applied foams, one and two component froth foams, insulation for refrigerators and freezers, and in so called integral skin for applications such as steering wheels and other automotive or aerospace cabin parts, shoe soles, amusement park restraints and the like. The large-scale commercial acceptance of polyurethane foams is linked to the ability to provide a good balance of properties. For example, commercially valuable rigid polyurethane and polyisocyanurate foams provide outstanding thermal insulation, excellent fire resistance properties, and superior structural properties at reasonably low densities. Integral skin foams should provide a tough durable outer skin and a cellular, cushioning core.

It is known in the art to produce rigid or semi-rigid polyurethane and polyisocyanurate foams by reacting a polyisocyanate with one or more polyols in the presence of one or more blowing agents, one or more catalysts, one or more surfactants and optionally other ingredients. Many different types of materials have been used as blowing agents, including certain hydrocarbons, fluorocarbons, chlorocarbons, chlorofluorocarbons, hydrochlorofluorocarbons, halogenated hydrocarbons, ethers, esters, aldehydes, alcohols, ketones, organic acids or other gas generating materials. The most commonly used gas generating material is water, which reacts with the polymeric MDI to generate CO₂. Heat is generated when the polyisocyanate reacts with the polyol, and volatilizes the blowing agent contained in the liquid mixture, thereby forming bubbles therein. In the case of gas generating materials, gaseous species are generated by thermal decomposition or reaction with one or more of the ingredients used to produce the polyurethane or polyisocyanurate foam. As the polymerization reaction proceeds, the liquid mixture becomes a cellular solid, entrapping the blowing agent in the cells of the foam. In some formulations, if a surfactant is not used in the foaming composition, the bubbles tend to pass through the liquid mixture without forming a foam or forming a foam with large, irregular cells rendering it less useful than desired for many applications.

The foam industry has historically used as blowing agents certain liquid fluorocarbons because of their ease of use and ability to produce foams with superior mechanical and thermal insulation properties. Certain fluorocarbons not only act as blowing agents by virtue of their volatility, but also are encapsulated or entrained in the closed cell structure of the rigid foam and are the major contributor to the advantageous low thermal conductivity properties, commonly called k-factor or lambda value of the rigid urethane foams. The k-factor is the rate of transfer of heat energy by conduction through one square foot of one-inch thick homogenous material in one hour where there is a difference of one degree Fahrenheit perpendicularly across the two surfaces of the material. Since the utility of closed-cell polyurethane-type foams is based, in part, on their thermal insulation properties, it would be advantageous to identify materials that produce lower k-factor foams.

Preferred blowing agents also have low global warming potential. Among these are certain hydrohaloolefins including hydrofluoroolefins of which trans-1,3,3,3-tetrafluoropropene (1234ze(E)) and 1,1,1,4,4,4hexafluorobut-2-ene (1336mzzm(Z)) are of particular interest and hydrochlorofluoroolefins of which 1-chloro-3,3,3-trifluoropropene (1233zd) (including both cis and trans isomers and combinations thereof) is of particular interest. Processes for the manufacture of trans-1,3,3,3-tetrafluoropropene are disclosed in U.S. patents 7,230,146 and 7,189, 884. Processes for the manufacture of trans-1-chloro-3,3,3-trifluoropropene are disclosed in U.S. patents 6,844,475 and 6,403,847.

It is convenient in many applications to provide the components for polyurethane or polyisocyanurate foams in pre-blended formulations. Typically, the foam formulation is pre-blended into two components. The polyisocyanate and optionally isocyanate compatible raw materials, including but not limited to certain blowing agents and non-reactive surfactants, comprise the first component, commonly referred to as the "A" component. A polyol or mixture of polyols, one or more surfactant, one or more catalyst, one or more blowing agent, and other optional components including but not limited to flame retardants, colorants, compatibilizers, and solubilizers comprise the second component, commonly referred to as the "B" component. Accordingly, polyurethane or polyisocyanurate foams are readily prepared by bringing together the A and B side components either by hand mix for small preparations and, preferably, machine mix techniques to form blocks, slabs, laminates, pour-in-place panels and other items, spray applied foams, froths, and the like. Optionally, other ingredients such as fire retardants, colorants, auxiliary blowing agents, and other polyols can be added to the mixing head or reaction site. Most conveniently, however, they are all incorporated into one B component.

Applicants have come to appreciate that a shortcoming of two-component systems, especially those using certain hydrohaloolefins, including 1234ze(E), 1336mzzm(Z), and 1233zd(E), is the shelf-life of the B-side composition. Normally when a foam is produced by bringing together the A and B side components, a good foam is obtained. However, applicants have found that if the polyol premix composition containing a halogenated olefin blowing agent and a typical amine-containing catalyst is aged, prior to treatment with the polyisocyanate, deleterious effects can occur. For example, applicants have found that such formulations can produce a foamable composition which has an undesirable increase in reactivity time and/or a subsequent cell coalescence. The resulting foams are of lower quality and/or may even collapse during the formation of the foam, leading to poor foam structure.

### SUMMARY

One aspect of the present invention provides blowing agent compositions, foamable compositions and foaming methods which comprise a hydrohaloolefin component, surfactant and at least one adsorbent, said adsorbent preferably being of a type effective and present in an amount effective to reduce, and preferably substantially reduce, the content of halogen ion in the composition. Although applicants do not intend to be bound by or limited to any particular theory of operation, applicants have observed and determined that the decreased shelf stability of certain polyol premix compositions originates, at least in part, from the reaction of certain amine catalysts with certain hydrohaloolefins which releases halogen ions into the composition. Applicants have determined that the presence of such halogen ions in the composition or portion of the composition which contains the surfactant, and particularly the polymeric silicone surfactant, has a negative impact on the ability of the surfactant to achieve the desired function, particularly by decreasing the molecular weight of the polymeric silicone surfactant(s), present in the composition. While it is possible to solve the problem by separating the blowing agent, surfactant, and catalyst, for example by adding the blowing agent, amine catalyst, or surfactant to the polyisocyanate, ("A" component) or by introducing the blowing agent, amine catalyst, or surfactant using a separate stream from the "A" or "B" component, a preferred solution is one that does not require a change in the way the foams are typically made. Applicants have discovered that the addition of certain adsorbent agents or materials can be used to effectively reduce the content of the halogen ions in the composition to the point of substantially reducing, and preferably substantially eliminating, the problem of surfactant degradation and loss of function. Accordingly, the inclusion of such agents in accordance with the teachings of the present invention results in the ability to produce good quality foams even if the components of the polyol blend have been maintained together, such as would occur in storage for example, for several weeks or months. The preferred resulting foams, and particularly the preferred semi-rigid, polyurethane and polyisocyanurate foams, are characterized by a fine uniform cell structure and little or no measurable foam collapse.

In certain embodiments, the present invention relates to polyol premix compositions including an absorbing agent with a combination of at least one blowing agent, a polyol, at least one surfactant (including a silicone and/or non-silicone surfactant), and a catalyst (including an amine and/or non-amine catalyst), wherein the blowing agent comprises a hydrohaloolefin, and optionally a hydrocarbon, fluorocarbon, chlorocarbon, fluorochlorocarbon, halogenated hydrocarbon, CO₂ generating material, or combinations thereof.

In certain broad aspects of the present invention, it is contemplated that the adsorbing agent may be any material that substantially reduces and preferably substantially eliminates the ability of the halogen ions (e.g. fluoride ions) to degrade the silicone surfactant, and/or measurably enhances the shelf-life of the composition relative to the same composition without the adsorbing agent. In certain embodiments, the adsorbent material is a halogen ion scavenger, which may include, but is not limited to, a silica gel, fumed silica, activated charcoal, calcium sulfate, calcium chloride, montmorillonite clay, a molecular sieve, or combinations thereof. In further embodiments, the halogen ion scavenger is a molecular sieve having a pore diameter adapted to absorb the halogen ions, particularly fluoride ions. Such a pore diameter may include a diameter within the range of about 3Å to about 10 Å, in further embodiments between about 3Å to about 8 Å, and in even further embodiments between about 5Å to about 8 Å.

The adsorbing materials are provided in an effective amount, which may be any amount to accomplish the advantages provided herein or as otherwise defined herein. In certain embodiments, the adsorbing material is provided in an amount between about 0.1 wt. % and about 20 wt. %, between about 0.1 wt. % and about 10 wt. %, between about 0.3 wt. % and about 8 wt. %, or between about 0.5 wt. % and about 5 wt. %, based on the total weight of the components that will be maintained together in the composition prior to use.

The inventive hydrohaloolefin includes 1,3,3,3-tetrafluoropropene, 1-chloro-3,3,3-trifluoropropene, 1,1,1,4,4,4hexafluorobut-2-ene or stereoisomers thereof, and combinations of any two or more thereof.

Additional advantages and embodiments of the present invention will be readily apparent to one of skill of the art, based on the disclosure and Examples provided herein.

### DETAILED DESCRIPTION OF THE INVENTION

The present invention relates, in part, to the use of adsorbent materials in blowing agents foamable compositions and foams to improve the effectiveness of the composition, especially the effectiveness of the composition wherein there will be a substantial time period after the composition is formed but prior to use, which in preferred embodiments enhances the storage stability of the composition. While not necessarily limiting to the invention, and without being bound by or to any particular theory of operation, it is believed that in polyol premix compositions using a hydrohaloolefin blowing agent and an amine catalyst that the amine catalyst reacts with the blowing agent to produce halogen ions, such as fluoride ions. Such a reaction is believed to lead to a decrease in the reactivity of the blowing agent. It is further believed that the halogen ion, such as fluoride ion, reacts with silicone surfactant present in such compositions to produce a lower average molecular weight surfactant, which is less effective than originally intended. Such depletion/degradation within the composition is believed to result in a foaming process that results in a reduced integrity of the foam cell wall and/or shape and/or size of the cell and, hence, produce a foam that is subject to higher than desired levels of cell collapse.

One aspect of the present invention provides polyol premix compositions which include a combination of one or more adsorbent materials or agents with at least one haloolefin blowing agent, one or more polyols, one or more surfactants (include silicone and/or non-silicone surfactant), and one or more catalysts (including amine and/or non-amine catalysts). In one non-limiting embodiment, the adsorbent material(s) may be any material that reduces and preferably substantially reduces, and even more preferably substantially removes halogen ions from the ability to negatively interact with surfactant in the composition. In certain embodiments, such adsorbent material(s) substantially removes or is adapted to substantially remove fluoride ions from the ability to negatively interact with surfactant in the composition, such as those ions produced through the reaction of the haloolefin blowing agent with an amine catalyst(s). In certain embodiments, the adsorbent material(s) substantially and/or measurably reduce degradation of at least the silicone surfactant and/or measurably/substantially enhance the shelf-life of the composition.

The adsorbent material is a halogen ion scavenger, which includes silica gel, fumed silica, activated charcoal, calcium, chloride, montmorillonite clay, a molecular sieve, or combinations thereof. In further embodiments, the halogen ion scavenger is a molecular sieve having a specific pore diameter adapted to absorb the halogen ions, particularly fluoride ions. Such a pore diameter may include, but are not limited to, a diameter within the range of about 3Å to about 10 A, in further embodiments between about 3Å to about 8 Å, or between about 5 Å to about 8 Å. Embodiments of such molecular sieves include, but are not limited to Type 5A - Type 13X molecular sieves, such as those produced by UOP, LLC.

The adsorbing materials are preferably provided to the composition in an effective amount. As used herein, the term "effective amount," as it relates to the adsorbing materials, means any amount that removes sufficient halogen ions (e.g. fluoride ions) from the composition to enhance the stability of the composition as described above. Preferably, the "effective amount" is sufficient to substantially reduce degradation of at least the silicone surfactant and/or substantially or otherwise measurably improves the shelf-life of the composition. In certain preferred embodiments, the type and amount of adsorbing material according to the present invention is effective to provide an Accelerated Aging Delay (also sometimes referred to herein as AGD) in cell collapse of at least one (1) day, more preferably at least two (2) days and even more preferably at least about five (5) days. As used herein, AGD means the delay in cell collapse that occurs according to the accelerated aging tests in the Examples, in comparison to the same formulation without the adsorbing material. In certain embodiments, the adsorbing material is provided in an amount between about 0.1 wt. % and about 20 wt. %, between about 0.1 wt. % and about 10 wt. %, between about 0.3 wt. % and about 8 wt. %, or between about 0.5 wt. % and about 5 wt. % of the composition, based on the total weight of the components that are present together in the composition prior to use. The polyol premix is blended thoroughly with such amounts of the adsorbent materials for optimal performance. The adsorbent material can be blended in the polyol premix during the polyol premix blending process and stored within premix in standard storage drums or containers for storage and/or delivery to customers.

### A. THE HYDROHALOOLEFIN BLOWING AGENT(S)

The hydrohaloolefin blowing agent may include at least one halooalkene such as a fluoroalkene or chlorofluoroalkene containing from 3 to 4 carbon atoms and at least one carbon-carbon double bond. Preferred hydrohaloolefins non-exclusively include trifluoropropenes, tetrafluoropropenes such as (1234), pentafluoropropenes such as (1225), chlorotrifloropropenes such as (1233), chlorodifluoropropenes, chlorotrifluoropropenes, chlorotetrafluoropropenes, hexafluorobutenes (1336) and combinations of these. Disclosed are the tetrafluoropropene, pentafluoropropene, and chlorotrifloropropene compounds in which the unsaturated terminal carbon has not more than one F or Cl substituent. Included are 1,3,3,3-tetrafluoropropene (1234ze); 1,1,3,3-tetrafluoropropene; 1,2,3,3,3-pentafluoropropene (1225ye), 1,1,1-trifluoropropene; 1,2,3,3,3-pentafluoropropene, 1,1,1,3,3-pentafluoropropene (1225zc) and 1,1,2,3,3-pentafluoropropene (1225yc); (Z)-1,1,1,2,3-pentafluoropropene (1225yez); 1-chloro-3,3,3-trifluoropropene (1233zd), 1,1,1,4,4,4-hexafluorobut-2-ene (1336mzzm) or combinations thereof, and any and all stereoisomers of each of these. Additional blowing agents that may be used alone or in combination with the foregoing are provided in U.S. Published Patent Application No. 2009/0253820, including but not limited to pages 3-5.

Preferred hydrohaloolefins have a Global Warming Potential (GWP) of not greater than 150, more preferably not greater than 100 and even more preferably not greater than 75. As used herein, "GWP" is measured relative to that of carbon dioxide and over a 100-year time horizon, as defined in "The Scientific Assessment of Ozone Depletion, 2002, a report of the World Meteorological Association's Global Ozone Research and Monitoring Project,". Preferred hydrohaloolefins also preferably have an Ozone Depletion Potential (ODP) of not greater than 0.05, more preferably not greater than 0.02 and even more preferably about zero. As used herein, "ODP" is as defined in "The Scientific Assessment of Ozone Depletion, 2002, A report of the World Meteorological Association's Global Ozone Research and Monitoring Project," which is incorporated herein by reference.

In certain aspects, the hydrohaloolefin blowing agent component includes at least one or a combination of 1234ze(E), 1233zd(E), and isomer blends thereof and/or 1336mzzm(Z), and optionally a hydrocarbon, fluorocarbon, chlorocarbon, fluorochlorocarbon, halogenated hydrocarbon, ether, fluorinated ether, ester, alcohol, aldehyde, ketone, organic acid, gas generating material, water or combinations thereof.

Hydrohaloolefin blowing agent compositions of the present invention may also include one or more optional co-blowing agents. In certain, non-limiting aspect, such co-blowing agents include water, organic acids that produce CO₂ and/or CO, hydrocarbons; ethers, halogenated ethers; esters, alcohols, aldehydes, ketones, pentafluorobutane; pentafluoropropane; hexafluoropropane; heptafluoropropane; trans-1,2 dichloroethylene; methylal, methyl formate; 1-chloro-1,2,2,2-tetrafluoroethane (124); 1,1-dichloro-1-fluoroethane (141b); 1,1,1,2-tetrafluoroethane (134a); 1,1,2,2-tetrafluoroethane (134); 1-chloro 1,1-difluoroethane (142b); 1,1,1,3,3-pentafluorobutane (365mfc); 1,1,1,2,3,3,3-heptafluoropropane (227ea); trichlorofluoromethane (11); dichlorodifluoromethane (12); dichlorofluoromethane (22); 1,1,1,3,3,3-hexafluoropropane (236fa); 1,1,1,2,3,3-hexafluoropropane (236ea); 1,1,1,2,3,3,3-heptafluoropropane (227ea), difluoromethane (32); 1,1-difluoroethane (152a); 1,1,1,3,3-pentafluoropropane (245fa); butane; isobutane; normal pentane; isopentane; cyclopentane, or combinations thereof. In certain embodiments the co-blowing agent(s) include one or a combination of water and/or normal pentane, isopentane or cyclopentane, which may be provided with one or a combination of the hydrohaloolefin blowing agents discussed herein. Additional co-blowing agents may include any such blowing agents that are known or otherwise useable in a foam, foamable composition, or foam premix. Non-limiting examples of such co-blowing agents are provided in U.S. Published Patent Application No. 2009/0253820, including pages 5-7.

The blowing agent component is usually present in the polyol premix composition in an amount of from about 1 wt.% to about 30 wt.%, preferably from about 3 wt.% to about 25 wt.%, and more preferably from about 5 wt.% to about 25 wt.%, by weight of the polyol premix composition. When both a hydrohaloolefin and an optional blowing agent are present, the hydrohaloolefin component is usually present in the blowing agent component in an amount of from about 5 wt.% to about 90 wt.%, preferably from about 7 wt.% to about 80 wt.%, and more preferably from about 10 wt.% to about 70 wt.%, by weight of the blowing agent component; and the optional blowing agent is usually present in the blowing agent component in an amount of from about 95 wt.% to about 10 wt.%, preferably from about 93 wt.% to about 20 wt.%, and more preferably from about 90 wt.% to about 30 wt.%, by weight of the blowing agent component.

### B. THE POLYOL COMPONENT

The polyol component, which includes mixtures of polyols, can be any polyol which reacts in a known fashion with an isocyanate in preparing a polyurethane or polyisocyanurate foam. Useful polyols comprise one or more of a sucrose containing polyol; phenol, a phenol formaldehyde containing polyol; a glucose containing polyol; a sorbitol containing polyol; a methylglucoside containing polyol; an aromatic polyester polyol; glycerol; ethylene glycol; diethylene glycol; propylene glycol; graft copolymers of polyether polyols with a vinyl polymer; a copolymer of a polyether polyol with a polyurea; one or more of (a) condensed with one or more of (b), wherein (a) is selected from glycerine, ethylene glycol, diethylene glycol, trimethylolpropane, ethylene diamine, pentaerythritol, soy oil, lecithin, tall oil, palm oil, and castor oil; and (b) is selected from ethylene oxide, propylene oxide, a mixture of ethylene oxide and propylene oxide; and combinations thereof. The polyol component is usually present in the polyol premix composition in an amount of from about 60 wt.% to about 95 wt.%, preferably from about 65 wt.% to about 95 wt.%, and more preferably from about 70 wt.% to about 90 wt.%, by weight of the polyol premix composition.

### C. SURFACTANT

In certain aspects, the polyol premix composition contains a silicone surfactant. The silicone surfactant is used to aid in the formation of foam from the mixture, as well as to influence or control the size of the bubbles of the foam so that a foam of a desired cell structure is obtained. In many embodiments, a foam with small bubbles or cells therein of uniform size is desired since it has the most desirable physical properties such as compressive strength and thermal conductivity. Also, it is desirable to have a foam with stable cells which do not collapse prior to forming or during foam rise.

Silicone surfactants generally are adaptable for use in the preparation of polyurethane or polyisocyanurate foams of the present invention, and many such surfactants are available under a number of trade names known to those skilled in this art. Such materials have been found to be applicable over a wide range of formulations allowing uniform cell formation and maximum gas entrapment to achieve very low density foam structures. The preferred silicone surfactant comprises a polysiloxane polyoxyalkylene block co-polymer. Some representative silicone surfactants useful for this invention are Momentive's L-5130, L-5180, L-5340, L-5440, L-6100, L-6900, L-6980 and L-6988; Air Products DC-193, DC-197, DC-5582 , and DC-5598; and B-8404, B-8407, B-8409 and B-8462 from Evonik Industries AG of Essen, Germany. Others are disclosed in U.S. patents 2,834,748; 2,917,480; 2,846,458 and 4,147,847. The silicone surfactant component is usually present in the polyol premix composition in an amount of from about 0.5 wt.% to about 5.0 wt.%, preferably from about 1.0 wt.% to about 4.0 wt.%, and more preferably from about 1.5 wt.% to about 3.0 wt.%, by weight of the polyol premix composition.

The polyol premix composition may also/or alternatively contain a non-silicone surfactant. Useful non-silicone surfactants include non-ionic non-silicone surfactants, anionic non-silicone surfactants, cationic non-silicone surfactants, ampholytic non-silicone surfactants, semi-polar non-silicone surfactants, zwitterionic non-silicone surfactants, and combinations thereof. Such surfactants include, but are not limited to, those described in U.S. Published Application No. 2009/0099273, the contents of which are incorporated herein by reference.

Useful non-silicone surfactants for use in the preparation of polyurethane or polyisocyanurate foams are available under a number of trade names known to those skilled in this art. Such materials have been found to be applicable over a wide range of formulations allowing uniform cell formation and maximum gas entrapment to achieve very low density foam structures. A preferred non-silicone non-ionic surfactant is LK-443 which is commercially available from Air Products Corporation.

### D. THE CATALYST SYSTEM

The inventive polyol premix composition next contains a catalyst system. In one aspect, such amines have the formula R₁R₂N-[A-NR₃]ₙR₄ wherein each of R₁, R₂, R₃, and R₄ is independently H, a C₁ to C₈ alkyl group, a C₁ to C₈ alkenyl group, a C₁ to C₈ alcohol group, or a C₁ to C₈ ether group, or R₁ and R₂ together form a C₅ to C₇ cyclic alkyl group, a C₅ to C₇ cyclic alkenyl group, a C₅ to C₇ heterocyclic alkyl group, or a C₅ to C₇ heterocyclic alkenyl group; A is a C₁ to C₅ alkyl group, a C₁ to C₅ alkenyl group, or an ether; n is 0, 1, 2, or 3. In further embodiments, the catalyst system includes an amine catalyst that includes sterically hindered amines and/or adducts of an amine and an organic acid.

Useful amines include a primary amine, secondary amine or tertiary amine. Useful tertiary amine catalysts non-exclusively include dicyclohexylmethylamine; ethyldiisopropylamine; dimethylcyclohexylamine; dimethylisopropylamine; triethylenediamine, methylisopropylbenzylamine; methylcyclopentylbenzylamine; isopropyl-sec-butyl-trifluoroethylamine; diethyl-(α-phenylethyl)amine, tri-n-propylamine, or combinations thereof. Useful secondary amine catalysts non-exclusively include dicyclohexylamine; t-butylisopropylamine ; di-t-butylamine; cyclohexyl-t-butylamine; di-sec-butylamine, dicyclopentylamine; di-(α-trifluoromethylethyl)amine; di-(α-phenylethyl)amine; or combinations thereof. Useful primary amine catalysts non-exclusively include: triphenylmethylamine and 1,1-diethyl-n-propylamine.

Other useful amines include morpholines, imidazoles, ether containing compounds, and the like. These include, but are not limited to, the following:
dimorpholinodiethylether
N-ethylmorpholine
N-methylmorpholine
bis(dimethylaminoethyl) ether
imidizole
n-methylimidazole
1,2-dimethylimidazole
dimorpholinodimethylether
N,N,N',N',N",N"-pentamethyldiethylenetriamine
N,N,N',N',N",N"-pentaethyldiethylenetriamine
N,N,N',N',N",N"-pentamethyldipropylenetriamine bis(diethylaminoethyl) ether
bis(dimethylaminopropyl) ether.

The amine catalyst is usually present in the polyol premix composition in an amount of from about 0.1 wt.% to about 3.5 wt.%, preferably from about 0.2 wt.% to about 3.0 wt.%, and more preferably from about 0.5 wt.% to about 2.5 wt.%, by weight of the polyol premix composition.

The polyol premix composition may optionally further comprise a non-amine catalyst, which may be used alone or in conjunction with an amine catalyst. In one embodiment, the non-amine catalysts are inorgano- or organo-metallic compounds. Useful inorgano- or organo-metallic compounds include, but are not limited to, organic salts, Lewis acid halides, or the like, of any metal, including, but not limited to, transition metals, post-transition (poor) metals, rare earth metals (e.g. lanthanides), metalloids, alkali metals, alkaline earth metals, or the like. Examples of such metals may include, but are not limited to, bismuth, lead, tin, zinc, chromium, cobalt, copper, iron, manganese, magnesium, potassium, sodium, titanium, mercury, zinc, antimony, uranium, cadmium, thorium, aluminum, nickel, cerium, molybdenum, vanadium, zirconium, or combinations thereof. Non-exclusive examples of such inorgano- or organo-metallic catalysts include, but are not limited to, bismuth nitrate, lead 2-ethylhexoate, lead benzoate, lead naphthanate, ferric chloride, antimony trichloride, antimony glycolate, tin salts of carboxylic acids, dialkyl tin salts of carboxylic acids, potassium acetate, potassium octoate, potassium 2-ethylhexoate, potassium salts of carboxylic acids, zinc salts of carboxylic acids, zinc 2-ethylhexanoate, glycine salts, alkali metal carboxylic acid salts, sodium N-(2-hydroxy-5-nonylphenol)methyl-N-methylglycinate, tin (II) 2-ethylhexanoate, dibutyltin dilaurate, or combinations thereof. These catalysts are usually present in the polyol premix composition in an amount of from about 0.25 wt.% to about 3.0 wt.%, preferably from about 0.3 wt.% to about 2.5 wt.%, and more preferably from about 0.35 wt.% to about 2.0 wt. %, by weight of the polyol premix composition. While these are usual amounts, the quantity amount of the foregoing catalyst can vary widely, and the appropriate amount can be easily be determined by those skilled in the art.

Furthermore, Applicants have found that it is desirable to use certain metal-based non-amine catalysts in foamable and foaming systems having relatively high levels of water, and particularly high-water polyol pre-mix compositions. More specifically, applicants have found that catalysts based on tin and potassium are preferred in such systems because of their ability to retain their reactivity and avoid stability problems in such high water systems. Furthermore, applicants have found that certain catalysts based upon zinc and bismuth have acceptable performance in systems having relatively low water content but do not produce acceptable results in high-water content systems and compositions. Applicants have found that the class of metal catalysts described above, and preferably zinc-based catalysts, and preferably in certain embodiments amine/zinc-based catalyst blends are capable of performing effectively in high-water content systems and compositions wherein the catalyst is a precipitation-resistant metal-based catalyst(s) as that term is defined herein. In other or additional embodiments, applicants have found that it is preferred in certain systems that the metal catalysts comprise at least a first catalysts based upon tin and/or zinc, and a second catalyst based upon potassium and/or bismuth.

In another embodiment of the invention, the non-amine catalyst is a quaternary ammonium carboxylate. Useful quaternary ammonium carboxylates include, but are not limited to: (2-hydroxypropyl)trimethylammonium 2-ethylhexanoate (TMR^{®} sold by Air Products and Chemicals) and (2-hydroxypropyl)trimethylammonium formate (TMR-2^{®} sold by Air Products and Chemicals). These quaternary ammonium carboxylate catalysts are usually present in the polyol premix composition in an amount of from about 0.25 wt.% to about 3.0 wt.%, preferably from about 0.3 wt.% to about 2.5 wt.%, and more preferably from about 0.35 wt.% to about 2.0 wt. %, by weight of the polyol premix composition. While these are usual amounts, the quantity amount of catalyst can vary widely, and the appropriate amount can be easily be determined by those skilled in the art.

In embodiments where an amine catalyst is provided, the catalyst may be provided in any amount to achieve the function of the instant invention without affecting the foam forming or storage stability of the composition, as characterized herein. To this end, the amine catalyst may be provided in amounts less than or greater than the non-amine catalyst.

The present invention is not limited to the use of the foregoing catalysts systems and may also include other catalysts systems known in the art, including those used, or which may be used, in accordance with the foam, foamable compositions, or foam premixes discussed herein. Non-limiting examples of such catalysts systems may be found in one or more of the following U.S. Published Patent Applications: U.S. 2009/0099272, U.S. 2009/0099273, U.S. 2009/0099274, U.S. 2011/0152392, U.S. 2012/0220677, U.S. 2012/0248371, and U.S. 2012/031303 5.

### E. FOAM PREPARATION

The preparation of polyurethane or polyisocyanurate foams using the compositions described herein may follow any of the methods well known in the art can be employed, see Saunders and Frisch, Volumes I and II Polyurethanes Chemistry and technology, 1962, John Wiley and Sons, New York, N.Y. or Gum, Reese, Ulrich, Reaction Polymers, 1992, Oxford University Press, New York, N.Y. or Klempner and Sendijarevic, Polymeric Foams and Foam Technology, 2004, Hanser Gardner Publications, Cincinnati, OH. In general, polyurethane or polyisocyanurate foams are prepared by combining an isocyanate, the polyol premix composition, and other materials such as optional flame retardants, colorants, or other additives. These foams can be rigid, flexible, or semi-rigid, and can have a closed cell structure, an open cell structure or a mixture of open and closed cells.

It is convenient in many applications to provide the components for polyurethane or polyisocyanurate foams in pre-blended formulations. Most typically, the foam formulation is pre-blended into two components. The isocyanate and optionally other isocyanate compatible raw materials, including but not limited to blowing agents and certain silicone surfactants, comprise the first component, commonly referred to as the "A" component. The polyol mixture composition, including surfactant, catalysts, blowing agents, absorbing agents/materials and optional other ingredients comprise the second component, commonly referred to as the "B" component. In any given application, the "B" component may not contain all the above listed components, for example some formulations omit the flame retardant if flame retardancy is not a required foam property. Accordingly, polyurethane or polyisocyanurate foams are readily prepared by bringing together the A and B side components either by hand mix for small preparations and, preferably, machine mix techniques to form blocks, slabs, laminates, pour-in-place panels and other items, spray applied foams, froths, and the like. Optionally, other ingredients such as fire retardants, colorants, auxiliary blowing agents, water, and even other polyols can be added as a stream to the mix head or reaction site. Most conveniently, however, they are all incorporated into one B component as described above.

A foamable composition suitable for forming a polyurethane or polyisocyanurate foam may be formed by reacting an organic polyisocyanate and the polyol premix composition described above. Any organic polyisocyanate can be employed in polyurethane or polyisocyanurate foam synthesis inclusive of aliphatic and aromatic polyisocyanates. Suitable organic polyisocyanates include aliphatic, cycloaliphatic, araliphatic, aromatic, and heterocyclic isocyanates which are well known in the field of polyurethane chemistry. These are described in, for example, U.S. patents 4,868,224; 3,401,190; 3,454,606; 3,277,138; 3,492,330; 3,001,973; 3,394,164; 3,124.605; and 3,201,372. Preferred as a class are the aromatic polyisocyanates.

Representative organic polyisocyanates correspond to the formula:

R(NCO)z

wherein R is a polyvalent organic radical which is either aliphatic, aralkyl, aromatic or mixtures thereof, and z is an integer which corresponds to the valence of R and is at least two. Representative of the organic polyisocyanates contemplated herein includes, for example, the aromatic diisocyanates such as 2,4-toluene diisocyanate, 2,6-toluene diisocyanate, mixtures of 2,4- and 2,6-toluene diisocyanate, crude toluene diisocyanate, methylene diphenyl diisocyanate, crude methylene diphenyl diisocyanate and the like; the aromatic triisocyanates such as 4,4',4"-triphenylmethane triisocyanate, 2,4,6-toluene triisocyanates; the aromatic tetraisocyanates such as 4,4'-dimethyldiphenylmethane-2,2'5,5-'tetraisocyanate, and the like; arylalkyl polyisocyanates such as xylylene diisocyanate; aliphatic polyisocyanate such as hexamethylene-1,6-diisocyanate, lysine diisocyanate methylester and the like; and mixtures thereof. Other organic polyisocyanates include polymethylene polyphenylisocyanate, hydrogenated methylene diphenylisocyanate, m-phenylene diisocyanate, naphthylene-1,5-diisocyanate, 1-methoxyphenylene-2,4-diisocyanate, 4,4'-biphenylene diisocyanate, 3,3'-dimethoxy-4,4'-biphenyl diisocyanate, 3,3'-dimethyl-4,4'-biphenyl diisocyanate, and 3,3'-dimethyldiphenylmethane-4,4'-diisocyanate; Typical aliphatic polyisocyanates are alkylene diisocyanates such as trimethylene diisocyanate, tetramethylene diisocyanate, and hexamethylene diisocyanate, isophorene diisocyanate, 4, 4'-methylenebis(cyclohexyl isocyanate), and the like; typical aromatic polyisocyanates include m-, and p-phenylene disocyanate, polymethylene polyphenyl isocyanate, 2,4- and 2,6-toluenediisocyanate, dianisidine diisocyanate, bitoylene isocyanate, naphthylene 1,4-diisocyanate, bis(4-isocyanatophenyl)methene, bis(2-methyl-4-isocyanatophenyl)methane, and the like. Preferred polyisocyanates are the polymethylene polyphenyl isocyanates, Particularly the mixtures containing from about 30 to about 85 percent by weight of methylenebis(phenyl isocyanate) with the remainder of the mixture comprising the polymethylene polyphenyl polyisocyanates of functionality higher than 2. These polyisocyanates are prepared by conventional methods known in the art. In the present invention, the polyisocyanate and the polyol are employed in amounts which will yield an NCO/OH stoichiometric ratio in a range of from about 0.9 to about 5.0. In the present invention, the NCO/OH equivalent ratio is, preferably, about 1.0 or more and about 3.0 or less, with the ideal range being from about 1.1 to about 2.5. Especially suitable organic polyisocyanate include polymethylene polyphenyl isocyanate, methylenebis(phenyl isocyanate), toluene diisocyanates, or combinations thereof.

In the preparation of polyisocyanurate foams, trimerization catalysts are used for the purpose of converting the blends in conjunction with excess A component to polyisocyanurate-polyurethane foams. The trimerization catalysts employed can be any catalyst known to one skilled in the art, including, but not limited to, glycine salts, tertiary amine trimerization catalysts, quaternary ammonium carboxylates, and alkali metal carboxylic acid salts and mixtures of the various types of catalysts. Preferred species within the classes are potassium acetate, potassium octoate, and sodium N-(2-hydroxy-5-nonylphenol)methyl-N-methylglycinate.

Conventional flame retardants can also be incorporated, preferably in amount of not more than about 20 percent by weight of the reactants. Optional flame retardants include tris(2-chloroethyl)phosphate, tris(2-chloropropyl)phosphate, tris(2,3-dibromopropyl)phosphate, tris(1,3-dichloropropyl)phosphate, tri(2-chloroisopropyl)phosphate, tricresyl phosphate, tri(2,2-dichloroisopropyl)phosphate, diethyl N,N-bis(2-hydroxyethyl) aminomethylphosphonate, dimethyl methylphosphonate, tri(2,3-dibromopropyl)phosphate, tri(1,3-dichloropropyl)phosphate, and tetra-kis-(2-chloroethyl)ethylene diphosphate, triethylphosphate, diammonium phosphate, various halogenated aromatic compounds, antimony oxide, aluminum trihydrate, polyvinyl chloride, melamine, and the like. Other optional ingredients can include from 0 to about 7 percent water, which chemically reacts with the isocyanate to produce carbon dioxide. This carbon dioxide acts as an auxiliary blowing agent. Formic acid is also used to produce carbon dioxide by reacting with the isocyanate and is optionally added to the "B"component.

In addition to the previously described ingredients, other ingredients such as, dyes, fillers, pigments and the like can be included in the preparation of the foams. Dispersing agents and cell stabilizers can be incorporated into the present blends. Conventional fillers for use herein include, for example, aluminum silicate, calcium silicate, magnesium silicate, calcium carbonate, barium sulfate, calcium sulfate, glass fibers, carbon black and silica. The filler, if used, is normally present in an amount by weight ranging from about 5 parts to 100 parts per 100 parts of polyol. A pigment which can be used herein can be any conventional pigment such as titanium dioxide, zinc oxide, iron oxide, antimony oxide, chrome green, chrome yellow, iron blue siennas, molybdate oranges and organic pigments such as para reds, benzidine yellow, toluidine red, toners and phthalocyanines.

The polyurethane or polyisocyanurate foams produced can vary in density from about 0.5 pounds per cubic foot to about 60 pounds per cubic foot, preferably from about 1.0 to 20.0 pounds per cubic foot, and most preferably from about 1.5 to 6.0 pounds per cubic foot. The density obtained is a function of how much of the blowing agent or blowing agent mixture disclosed in this invention plus the amount of auxiliary blowing agent, such as water or other co-blowing agents is present in the A and / or B components, or alternatively added at the time the foam is prepared. These foams can be rigid, flexible, or semi-rigid foams, and can have a closed cell structure, an open cell structure or a mixture of open and closed cells. These foams are used in a variety of well known applications, including but not limited to thermal insulation, cushioning, flotation, packaging, adhesives, void filling, crafts and decorative, and shock absorption.

The following non-limiting examples serve to illustrate the invention.

### EXAMPLES

### Example 1 - Stability Evaluation of 1233zd with Molecular Sieves

A polyol premix was prepared with 1233zd and one of two different molecular sieves in an enclosed test tube. Polyol pre-blend formulations are listed in Table 1, below. A total of four tubes are prepared for the controls and for each type of molecular sieve. The molecular sieves evaluated were UOP MOLSIV™ 5A and UOP MOLSIV™ 13X from UOP, LLC. These molecular sieves are synthetic crystalline metal aluminosilicates which have within their crystal structure a very high surface are and a uniform pore system. UOP MOLSIV ™ 5A has the structure Mₓ[(AlO₂)ₓ(SIO₂)y].zH₂O where M= Ca, Na and adsorbs molecules with critical diameters up to 5 angstroms. UOP MOLSIV ™ 13X has the structure Mₓ[(AlO₂)ₓ(SIO₂)_{y}].zH₂O where M=Na and absorbs molecules with critical diameters up to 10 angstroms.

Except for the control, each test tube contains 5 wt% of molecular sieves. Together with the controls, test tubes were aged in a 130°F oven for specified periods of time. Foams were then prepared with corresponding amount of isocyanate as per Table 2, below, after 7 days, 9 days, 13 days and 15days aging at 130°F. The appearance of foams was examined. Results are summarized in Table 3, below.

**Table 1. Polyol Preblend Composition with Molecular Sieve**

| Component | Weight (%) |
|---|---|
| Voranol 490 Polyol | 44.13 |
| Voranol 270 Polyol | 44.13 |
| Niax L6900 Silicone Surfactant | 1.32 |
| Polycat 5 Catalyst (N,N,N',N',N",N"-pentamethyldiethylenediamine) | 1.06 |
| Water | 1.32 |
| 1233zd(E) | 8.03 |
| Total | 100.00 |
| Molecular Sieves | 5.00 |
| Grand Total | 105.00 |

**Table 2. Polyurethane Foam Preparation with Molecular Sieve**

| | Control | 5A | 13X |
|---|---|---|---|
| Polyol Preblend | 70.0 | 73.5 | 73.5 |
| Isocyanate M20S | 74.1 | 74.1 | 74.1 |
| Total | 144.1 | 147.6 | 147.6 |

**Table 3. Foam Appearance after Heat Aging at 130°F**

| Heat Aging Duration | Control | With UOP Molecular Sieves | |
|---|---|---|---|
| | | 5A | 13X |
| 07 Days | Intact | Intact | Intact |
| 09 Days | Collapsed | Intact | Intact |
| 13 Days | Collapsed | Intact | Intact |
| 15 Days | Collapsed | Start Collapsing | Start Collapsing |

### Example 2 - Stability Evaluation of 1234ze with Fumed Silica

A polyol premix was prepared with 1234ze(E) and fumed silica in an enclosed test tube. Polyol pre-blend formulations are listed below in Table 4. A total of six tubes are prepared with three of them without fumed silica as control and three of them with fumed silica for evaluations. The tubes for evaluations consist of 0.5 wt% of fumed silica. Together with the controls, these tubes were aged in a 130°F oven for different period of time. Foam was then prepared with corresponding amount of isocyanate as per Table 5 after 1 days, 2 days and 3 days aging at 130°F. The appearance of the foams was examined and results are summarized in Table 6.

**Table 4. Polyol Preblend Composition with Fumed Silica**

| Component | Weight (%) |
|---|---|
| Voranol 490 Polyol | 44.13 |
| Voranol 270 Polyol | 44.13 |
| Niax L6900 Silicone Surfactant | 1.32 |
| Polycat 5 Catalyst (N,N,N',N',N",N"-pentamethyldiethylenediamine) | 1.06 |
| Water | 1.32 |
| 1234ze(E) | 7.04 |
| Total | 100.00 |
| Fumed Silica | 0.50 |
| Grand Total | 100.50 |

**Table 5. Polyurethane Foam Preparation with Fumed Silica**

| | Control | With Fumed Silica |
|---|---|---|
| Polyol Preblend | 70.0 | 70.5 |
| Isocyanate M20S | 74.1 | 74.1 |
| Total | 144.1 | 144.6 |

**Table 6. Foam Appearance after Heat Aging at 130°F**

| Heat Aging Duration | Control | With 0.5wt% Fumed Silica |
|---|---|---|
| 01 Days | Intact | Intact |
| 02 Days | Collapsed | Intact |
| 03 Days | Collapsed | Start Collapsing |

## Claims

1. A polyol premix composition comprising at least one blowing agent, a polyol, at least one silicone surfactant, a catalyst, and an adsorbing agent, wherein the blowing agent comprises a hydrohaloolefin, and optionally a hydrocarbon, fluorocarbon, chlorocarbon, fluorochlorocarbon, halogenated hydrocarbon, CO₂ or other gas generating material, or combinations thereof;
wherein the hydrohaloolefin comprises 1,3,3,3-tetrafluoropropene, 1-chloro-3,3,3-trifluoropropene, 1,1,1,4,4,4-hexafluorobut-2-ene, or stereoisomers thereof, or combinations thereof and
wherein the adsorbing agent is a halogen ion scavenger selected from the group consisting of a silica gel, fumed silica, activated charcoal, calcium chloride, montmorillonite clay, a molecular sieve, and combinations thereof.

2. The polyol premix composition of claim 1 wherein the halogen ion scavenger comprises a molecular sieve having a pore diameter between about 3Å to about 8 Å.

3. The polyol premix composition of claim 1 wherein the halogen ion scavenger comprises a molecular sieve having a pore diameter between about 5Å to about 8 Å.

4. The polyol premix composition of any one of claims 1 to 3, wherein the adsorbing material is provided in an amount between 0.1 wt. % and 20 wt. %.

5. The polyol premix composition of any one of claims 1 to 3, wherein the adsorbing material is provided in an amount between 0.1 wt. % and 10 wt. %.

6. The polyol premix composition of any one of claims 1 to 3, wherein the adsorbing material is provided in an amount between 0.3 wt. % and 8 wt. %.

7. The polyol premix composition of any one of claims 1 to 3, wherein the adsorbing material is provided in an amount between 0.5 wt. % and 5 wt. %.

8. The polyol premix composition of any one of claims 1 to 7, wherein the blowing agent has a Global Warming Potential (GWP) of not greater than 150.

9. The polyol premix composition of any one of claims 1 to 7, wherein the blowing agent has an Ozone Depletion Potential (ODP) of not greater than 0.05.

10. The polyol premix composition of any one of claims 1 to 9 wherein the blowing agent is present in the polyol premix composition in an amount of from 1 to 30 wt%.

11. A method of forming polyol premix composition which comprises combining a blowing agent, a polyol, a silicone surfactant, a catalyst and an absorbing agent, wherein the blowing agent comprises a hydrohaloolefin, and optionally a hydrocarbon, fluorocarbon, chlorocarbon, fluorochlorocarbon, halogenated hydrocarbon, CO₂ generating material, or combinations thereof wherein the hydrohaloolefin comprises 1,3,3,3-tetrafluoropropene, 1-chloro-3,3,3-trifluoropropene, 1,1,1,4,4,4-hexafluorobut-2-ene, or stereoisomers thereof, or combinations thereof; and
wherein the adsorbing agent is a halogen ion scavenger selected from the group consisting of a silica gel, fumed silica, activated charcoal, calcium chloride, montmorillonite clay, a molecular sieve, and combinations thereof.

12. A foamable composition comprising a mixture of an organic polyisocyanate and the polyol premix composition of any one of claims 1 to 10.

13. A polyurethane or polyisocyanurate foam produced from the foamable composition of claim 12.

14. The use of a polyurethane or polyisocyanurate foam of claim 13 in thermal insulation, cushioning, flotation, packaging, adhesives, void filling, crafts and decoration and shock absorption.

## Patentansprüche

1. Polyol-Premixzusammensetzung, umfassend mindestens ein Treibmittel, ein Polyol, mindestens ein Silikontensid, einen Katalysator und ein Adsorptionsmittel, wobei das Treibmittel ein Hydrohalogenolefin und gegebenenfalls einen Kohlenwasserstoff, einen Fluorkohlenwasserstoff, einen Chlorkohlenwasserstoff, einen Fluorchlorkohlenwasserstoff, einen halogenierten Kohlenwasserstoff, eine CO₂ oder ein anderes Gas erzeugende Substanz oder Kombinationen davon umfasst;
wobei das Hydrohalogenolefin 1,3,3,3-Tetrafluorpropen, 1-Chlor-3,3,3-trifluorpropen, 1,1,1,4,4,4-Hexafluorbut-2-en oder Stereoisomere davon oder Kombinationen davon umfasst und
wobei es sich bei dem Adsorptionsmittel um einen Halogenionenfänger aus der Gruppe bestehend aus einem Kieselgel, einer pyrogenen Kieselsäure, Aktivkohle, Calciumchlorid, Montmorillonit-Ton, einem Molsieb und Kombinationen davon handelt.

2. Polyol-Premixzusammensetzung nach Anspruch 1, wobei der Halogenionenfänger ein Molsieb mit einem Porendurchmesser zwischen etwa 3 Å bis etwa 8 Å umfasst.

3. Polyol-Premixzusammensetzung nach Anspruch 1, wobei der Halogenionenfänger ein Molsieb mit einem Porendurchmesser zwischen etwa 5 Å bis etwa 8 Å umfasst.

4. Polyol-Premixzusammensetzung nach einem der Ansprüche 1 bis 3, wobei das Adsorptionsmittel in einer Menge zwischen 0,1 Gew.-% und 20 Gew.-% bereitgestellt wird.

5. Polyol-Premixzusammensetzung nach einem der Ansprüche 1 bis 3, wobei das Adsorptionsmittel in einer Menge zwischen 0,1 Gew.-% und 10 Gew.-% bereitgestellt wird.

6. Polyol-Premixzusammensetzung nach einem der Ansprüche 1 bis 3, wobei das Adsorptionsmittel in einer Menge zwischen 0,3 Gew.-% und 8 Gew.-% bereitgestellt wird.

7. Polyol-Premixzusammensetzung nach einem der Ansprüche 1 bis 3, wobei das Adsorptionsmittel in einer Menge zwischen 0,5 Gew.-% und 5 Gew.-% bereitgestellt wird.

8. Polyol-Premixzusammensetzung nach einem der Ansprüche 1 bis 7, wobei das Treibmittel ein Treibhauspotential (Global Warming Potential, GWP) von nicht mehr als 150 aufweist.

9. Polyol-Premixzusammensetzung nach einem der Ansprüche 1 bis 7, wobei das Treibmittel ein Ozonschädigungspotential (Ozone Depletion Potential, ODP) von nicht mehr als 0,05 aufweist.

10. Polyol-Premixzusammensetzung nach einem der Ansprüche 1 bis 9, wobei das Treibmittel in der Polyol-Premixzusammensetzung in einer Menge von 1 bis 30 Gew.-% vorliegt.

11. Verfahren zur Bildung einer Polyol-Premixzusammensetzung, bei dem man ein Treibmittel, ein Polyol, ein Silikontensid, einen Katalysator und ein Adsorptionsmittel zusammengibt, wobei das Treibmittel ein Hydrohalogenolefin und gegebenenfalls einen Kohlenwasserstoff, einen Fluorkohlenwasserstoff, einen Chlorkohlenwasserstoff, einen Fluorchlorkohlenwasserstoff, einen halogenierten Kohlenwasserstoff, eine CO₂ oder ein anderes Gas erzeugende Substanz oder Kombinationen davon umfasst;
wobei das Hydrohalogenolefin 1,3,3,3-Tetrafluorpropen, 1-Chlor-3,3,3-trifluorpropen, 1,1,1,4,4,4-Hexafluorbut-2-en oder Stereoisomere davon oder Kombinationen davon umfasst und
wobei es sich bei dem Adsorptionsmittel um einen Halogenionenfänger aus der Gruppe bestehend aus einem Kieselgel, einer pyrogenen Kieselsäure, Aktivkohle, Calciumchlorid, Montmorillonit-Ton, einem Molsieb und Kombinationen davon handelt.

12. Verschäumbare Zusammensetzung, umfassend eine Mischung eines organischen Polyisocyanats und der Polyol-Premixzusammensetzung nach einem der Ansprüche 1 bis 10.

13. Polyurethan- oder Polyisocyanurat-Schaumstoff, hergestellt aus der verschäumbaren Zusammensetzung nach Anspruch 12.

14. Verwendung eines Polyurethan- oder Polyisocyanurat-Schaumstoffs nach Anspruch 13 bei der Wärmeisolierung, bei der Polsterung, bei der Flotation, bei der Verpackung, in Klebstoffen, bei der Hohlraumfüllung, im Handwerk und bei der Dekoration und Stoßdämpfung.

## Revendications

1. Composition de prémélange de polyol comprenant au moins un agent gonflant, un polyol, au moins un tensioactif de silicone, un catalyseur, et un agent adsorbant, dans laquelle l'agent gonflant comprend une hydrohalogéno-oléfine, et facultativement un hydrocarbure, fluorocarbone, chlorocarbone, fluorochlorocarbone, hydrocarbure halogéné, un matériau de génération de CO₂ ou d'un autre gaz, ou des combinaisons de ceux-ci ;
dans laquelle l'hydrohalogéno-oléfine comprend le 1,3,3,3-tétrafluoropropène, le 1-chloro-3,3,3-trifluoropropène, le 1,1,1,4,4,4-hexafluorobut-2-ène, ou des stéréoisomères de ceux-ci, ou des combinaisons de ceux-ci et
dans laquelle l'agent adsorbant est un piégeur d'ion halogène choisi dans le groupe constitué de gel de silice, silice pyrogénée, charbon actif, chlorure de calcium, argile montmorillonite, un tamis moléculaire et des combinaisons de ceux-ci.

2. Composition de prémélange de polyol de la revendication 1 dans laquelle le piégeur d'ion halogène comprend un tamis moléculaire ayant un diamètre de pore compris entre environ 3 Å et environ 8 Å.

3. Composition de prémélange de polyol de la revendication 1 dans laquelle le piégeur d'ion halogène comprend un tamis moléculaire ayant un diamètre de pore compris entre environ 5 Å et environ 8 Å.

4. Composition de prémélange de polyol de l'une quelconque des revendications 1 à 3, dans laquelle le matériau adsorbant est fourni dans une quantité comprise entre 0,1 % en poids et 20 % en poids.

5. Composition de prémélange de polyol de l'une quelconque des revendications 1 à 3, dans laquelle le matériau adsorbant est fourni dans une quantité comprise entre 0,1 % en poids et 10 % en poids.

6. Composition de prémélange de polyol de l'une quelconque des revendications 1 à 3, dans laquelle le matériau adsorbant est fourni dans une quantité comprise entre 0,3 % en poids et 8 % en poids.

7. Composition de prémélange de polyol de l'une quelconque des revendications 1 à 3, dans laquelle le matériau adsorbant est fourni dans une quantité comprise entre 0,5 % en poids et 5 % en poids.

8. Composition de prémélange de polyol de l'une quelconque des revendications 1 à 7, dans laquelle l'agent gonflant a un potentiel de réchauffement global (PRG) de pas plus de 150.

9. Composition de prémélange de polyol de l'une quelconque des revendications 1 boîte. En d'un texte À 7, dans laquelle l'agent gonflant a un potentiel de déplétion ozonique (PDO) de pas plus de 0,05.

10. Composition de prémélange de polyol de l'une quelconque des revendications 1 à 9 dans laquelle l'agent gonflant est présent dans la composition de prémélange de polyol dans une quantité de 1 à 30 % en poids.

11. Procédé de formation d'une composition de prémélange de polyol qui comprend la combinaison d'un agent gonflant, un polyol, un tensioactif de silicone, un catalyseur, et un agent adsorbant, dans lequel l'agent gonflant comprend une hydrohalogéno-oléfine, et facultativement un hydrocarbure, fluorocarbone, chlorocarbone, fluorochlorocarbone, hydrocarbure halogéné, un matériau de génération de CO₂ ou des combinaisons de ceux-ci
dans lequel l'hydrohalogéno-oléfine comprend le 1,3,3,3-tétrafluoropropène, le 1-chloro-3,3,3-trifluoropropène, le 1,1,1,4,4,4-hexafluorobut-2-ène, ou des stéréoisomères de ceux-ci, ou des combinaisons de ceux-ci ; et
dans lequel l'agent adsorbant est un piégeur d'ion halogène choisi dans le groupe constitué de gel de silice, silice pyrogénée, charbon actif, chlorure de calcium, argile montmorillonite, un tamis moléculaire et des combinaisons de ceux-ci.

12. Composition expansible comprenant un mélange d'un polyisocyanate organique et de la composition de prémélange de polyol de l'une quelconque des revendications 1 à 10.

13. Mousse de polyuréthane ou polyisocyanurate produite à partir de la composition expansible de la revendication 12.

14. Utilisation d'une mousse de polyuréthane ou polyisocyanurate de la revendication 13 dans l'isolation thermique, le rembourrage, la flottaison, l'emballage, les adhésifs, le remplissage de vide, l'artisanat et la décoration et l'absorption de choc.
